# EUROPÄISCHE PATENTSCHRIFT

(11) **EP 1 761 336 B1**
(45) Veröffentlichungstag und Bekanntmachung des Hinweises auf die Patenterteilung: **04.03.2020**
(21) Anmeldenummer: 05747818.2
(22) Anmeldetag: 10.06.2005
(51) Int. Cl.: B01J 37/00, B01J 37/08, B01J 29/40, B01J 35/00, C07D 487/08, C01B 39/00, B01J 35/02

(54) **VERFAHREN ZUR HERSTELLUNG EINES FORMKÖRPERS ENTHALTEND EIN MIKROPORÖSES MATERIAL UND MINDESTENS EIN SILICIUMHALTIGES BINDEMITTEL UND EIN VERFAHREN ZUR HERSTELLUNG VON TRIETHYLENDIAMIN (TEDA)**
METHOD FOR THE PRODUCTION OF A SHAPED BODY CONTAINING A MICROPOROUS MATERIAL AND AT LEAST ONE SILICON-CONTAINING BINDER AND A METHOD FOR PRODUCING TRIETHYLENEDIAMINE (TEDA)
PROCEDE DE PRODUCTION D'UN CORPS MOULE CONTENANT UN MATERIAU MICROPOREUX ET AU MOINS UN LIANT SILICEUX ET PROCEDE DE PRODUCTION DE TRIETHYLENDIAMINE (TEDA)

(30) Priorität: 18.06.2004 DE 102004029544
(43) Veröffentlichungstag der Anmeldung: 14.03.2007
(73) Patentinhaber: BASF SE, 67056 Ludwigshafen am Rhein (DE)
(72) Erfinder: BOSCH, Marco, 68159 Mannheim (DE); FRAUENKRON, Matthias, 67251 Freinsheim (DE); KOSTUR, Milan, 67063 Ludwigshafen (DE); HOFSTADT, Otto, 67346 Speyer (DE)
(74) Vertreter: BASF IP Association
(86) Internationale Anmeldenummer: PCT/EP2005/006242
(87) Internationale Veröffentlichungsnummer: WO 2005/123256

(56) Entgegenhaltungen:
- EP-A- 0 712 662
- EP-A- 0 952 152
- WO-A-01/02404
- WO-A-2005/053842
- DE-A1- 19 826 209
- US-A- 5 614 079
- US-A1- 2002 072 467

## Beschreibung

Die vorliegende Erfindung betrifft ein Verfahren zur Herstellung eines Formkörpers enthaltend ein zeolithisches Material, wobei das zeolithische Material ein Zeolith vom Pentasil Typ ist, und mindestens ein siliciumhaltiges Bindemittel, umfassend die Schritte
(I) Herstellen eines Gemischs, enthaltend das zeolithische Material und das Bindemittel, ein Anteigmittel und ein Lösungsmittel,
(II) Vermischen und Verdichten des Gemischs,
(III) Verformen des verdichteten Gemischs unter Erhalt eines Formkörpers,
(IV) Trocknen des Formkörpers und
(V) Calcinieren des getrockneten Formkörpers,
und ein Verfahren zur Herstellung von Triethylendiamin (TEDA) mit dem erfindungsgemäß hergestellten Katalysator.

TEDA (IUPAC-Name: 1,4-Diazabicyclo[2.2.2]-octan) ist ein wichtiges Zwischen- und Endprodukt in der chemischen Industrie, das hauptsächlich als solches bei der Polyurethanherstellung als Katalysator eingesetzt wird.

Zur Herstellung von Triethylendiamin (TEDA) existiert eine große Anzahl verschiedener Synthesen, die sich hauptsächlich in der Wahl der Edukte und der benutzten Katalysatoren unterscheiden.

Bekannt ist, dass TEDA durch eine Gasphasereaktion aus einer oder mehreren der Aminverbindungen Ethylendiamin (EDA), Monoethanolamin (MEOA), Diethanolamin, Triethanolamin, Piperazin (PIP), Diethylentriamin, Triethylentetramin, Tri(2-aminoethyl)amin, N-(2-aminoethyl)ethanolamin, Morpholin, N-(2-hydroxyethyl)piperazin, N,N'-Bis(2-hydroxyethyl)piperazin, N-(2-aminoethyl)piperazin bzw. N,N'-Bis(2-aminoethyl)piperazin an sauren heterogene Katalysatoren hergestellt werden kann.

Bei den kommerziellen Verfahren zur Herstellung von TEDA kann man zwischen zwei Katalysatortypen unterscheiden.

Bei dem klassischen Verfahren werden Alkali- und Erdalkaliphosphat-Katalysatoren eingesetzt. Alternativ kann ein zeolithkatalysiertes Verfahren benutzt werden.

Bei der Phosphatkatalyse werden hauptsächlich Piperazin, N-(2-hydroxyethyl)piperazin sowie N,N'-Bis(2-hydroxyethyl)piperazin umgesetzt. Die Selektivität an TEDA beträgt bis zu 85 % (Air Products, EP-A1-1 053 786).

Bei der Zeolithkatalyse werden hauptsächlich N-(2-aminoethyl)piperazin (Tosoh Corp., JP-B-3 132 061, JP-B-3 132 062, JP-B-3 132 063 und EP-A1-1 192 993), Ethylendiamin und/oder Piperazin (Air Products, EP-A1-842 936; BASF AG, EP-A1-382 055, WO 01/02404, EP-A1-1 215 211 und WO-A-03/004499) als Einsatzstoffe verwendet. Mit dieser Fahrweise können Selektivitäten an TEDA von bis zu 90 % erreicht werden.

Die ältere deutsche Patentanmeldung Nr. 10326137.0 vom 06.06.03 (BASF AG) betrifft ein Verfahren zur Erhöhung der Schneidhärte eines Formkörpers enthaltend ein kristallines Alumosilikat und chemische Syntheseverfahren in Gegenwart eines kristallinen Alumosilikat-Katalysators, hierunter insbesondere ein Verfahren zur Herstellung von Triethylendiamin (TEDA) durch Umsetzung von Ethylendiamin (EDA) und/oder Piperazin (PIP).

Zeolithe sind bekanntermaßen kristalline Alumosilikate mit geordneten Kanal- und Käfigstrukturen, die Mikroporen aufweisen, die vorzugsweise kleiner als ungefähr 0,9 nm sind. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W. M. Meier, D. H. Olson und Ch. Baerlocher, "Atlas of Zeolithe Structure Types", Elsevier, 5. Auflage, Amsterdam 2001.

Dabei sind im einzelnen etwa Zeolithe der Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen und insbesondere Zeolithe mit Pentasil-Struktur zu nennen.

Je nach Einsatzgebiet des Katalysators sollte oder muss dieser, neben der Kristallgröße, eine bestimmte Porengröße bzw. Porenverteilung aufweisen. Beispielsweise bei der Herstellung von Triethylendiamin hat sich der Einsatz eines zeolithischen Katalysators vom Strukturtyp ZSM-5 bewährt. Neben den vorstehend beschriebenen Eigenschaften sollte oder muss der Katalysator auch eine bestimmte chemische Zusammensetzung aufweisen. Im Falle des ZSM-5-Katalysators ist beispielsweise ein molares Verhältnis von Si zu Al im zeolithischen Material in bestimmten Bereichen vorteilhaft.

Die Verwendung eines Zeolithkatalysators vom Strukturtyp ZSM-5 bei der Herstellung von Triethylendiamin ist beispielsweise in der WO-A-01/02404 beschrieben, wobei das molare Verhältnis Si zu Al im Bereich von 100 bis 700 und besonders bevorzugt im Bereich von 150 bis 250 liegt.

Die WO-A-03/004499 beschreibt ein Verfahren zur selektiven Synthese von Triethylendiamin, bei der ein Zeolith-Katalysator, insbesondere vom ZSM-5-Typ, eingesetzt wird, der ein molares Verhältnis von Si zu einem Metall M im Bereich von größer 100, vorzugsweise von größer 200, weiter bevorzugt von größer 300 bis 40.000 und besonders bevorzugt von 400 bis 5000 aufweist. Dabei wird das Metall M, das in der Oxidationsstufe III bzw. IV auftreten kann, ausgewählt aus der Gruppe Al, B, Fe, Co, Ni, V, Mo, Mn, As, Sb, Bi, La, Ga, In, Y, Sc, Cr und Mischungen davon bzw. Ti, Zr, Ge, Hf, Sn und Mischungen davon. Besonders bevorzugt ist hierbei ein Alumosilikat.

Die EP-A1-1 215 211 beschreibt ein Verfahren zur Herstellung von Triethylendiamin, bei dem als Katalysator ein Zeolithkatalysator eingesetzt wird, der ein oder mehrere Metalle M in den Oxidationsstufen II, III oder IV als Oxide enthält, wobei im Falle, dass M gleich Al ist, das molare Verhältnis von SiO₂ zu Al₂O₃ größer als 1.400 ist. Neben ZSM-5 sind noch weitere Katalysatoren vom Strukturtyp ZSM-11, ZSM-23, ZSM-53, NU-87, ZSM-35 sowie Mischstrukturen offenbart.

Die ältere deutsche Patentanmeldung Nr. 10356184.6 vom 02.12.03 (BASF AG) betrifft ein zeolithisches Material vom Pentasil-Strukturtyp, insbesondere vom Strukturtyp ZSM-5, mit einem Alkali- und Erdalkaligehalt von höchstens 150 ppm und einem molaren Verhältnis von Si zu Al im Bereich von 250 bis 1500, wobei mindestens 95 Gew.-% der kugelförmigen Primärpartikel des zeolithischen Materials einen Durchmesser im Bereich von kleiner oder gleich 1 µm aufweisen und mindestens 95 % aller Primärpartikel kugelförmig sind. Ebenso betrifft diese Anmeldung einen Formkörper, der dieses zeolithische Material enthält sowie die Verwendung des zeolithischen Materials an sich oder des Formkörpers als Katalysator, insbesondere bei der Synthese von TEDA.

Die Verwendung von kollodialem Silica als SiO₂-Binder zur Herstellung von Katalysatorformkörpern ist in
"Catalyst Support and Supportes Catalysts" (A.B. Stiles), 1987, Kapitel 1, auf den Seiten 1 bis 9 und in Kapitel 3 auf den Seiten 57 bis 62, in "Applied Heterogeneous Catalysis - Design, Manufacture, Use Of Solid Catalysts" (J.-F. Lepage, J. Cosyns, P. Courty, E.B. Miller), 1987, Kapitel 5, auf den Seiten 75 bis 123, in "Heterogeneous Catalysis In Industrial Practice" (C.N. Satterfield), 2. Ausgabe, 1991, Kapitel 4, auf den Seiten 87 bis 130 und speziell auf Seite 121, sowie in
"Studies in Surface Science and Catalysis" (E.B.M. Doesburg, J.H.C. Hooff), 1993, Kapitel 8 auf den Seiten 309 bis 332,
beschrieben.

Der Einsatz von kollodialen Silica und speziell Ludox® AS40 der Firma DuPont als SiO₂-Binder für die Verformung von ZSM-5-Pulver wird in US 6,077,984 beschrieben.

Gemäß WO-A-01/23089 werden Katalysatorformkörper erhalten, bestehend aus einem ZSM-5-Pulver und einem SiO₂-Binder mit Schneidhärten größer 1 kg. Als SiO₂-Bindemittel werden kolloidale Silica eingesetzt.

In EP-A1-831 096 wird die Rolle des Bindemittels bei der katalytischen Herstellung von TEDA an ZSM-5-Katalysatoren beschrieben. Durch Verwendung einer Matrix (= Bindemittel) mit niedrigerer Acidität wie Silica und Zirconia sollen unerwünschte Nebenreaktionen unterdrückt werden.

In EP-A1-349 859 werden ZSM-5 Katalysatoren für die Herstellung von TEDA aus EDA/PIP-Gemischen beschrieben, die mit hochdispersem Silica als Bindemittel verformt sind. Als Hilfsstoffe für die Verstrangung können unter anderem Ethylcellulose, Stearinsäure, Kartoffelstärke und Silikoester verwendet werden.

EP-A1-1 192 993 betrifft ein Verfahren zur Herstellung von TEDA an Katalysatorformkörpern bestehend aus
a) einem kristallinen Alumosilikat mit einem molaren Verhältnis an Silica zu Alumina von mindestens 12 mit einem Anteil 30 bis 95 Gew.-%,
b) einem amorphen Silica mit einem Anteil von 5 bis 70 Gew.-%, und
c) eine Schneidhärte der Formkörper von mindestens 1 kg.

Die Verformung besteht darin, dass das amorphe Silica und das Alumosilikat in einem mechanischen Mischer vermischt und verdichtet werden, optional im Gegenwart von Wasser und die Formkörper durch Verformung in einem Verformungsgerät erhalten werden. Das amorphe Silica kennzeichnet sich durch Primärpartikel mit einem durchschnittlichen Durchmesser von 6 bis 60 nm aus.

Die Verwendung von siliciumhaltigen Verbindungen zur Passivierung der äußeren Zeolithoberfläche von ZSM-5-Katalysatoren und Verbesserung der Selektivität bei der Herstellung von TEDA wird in EP-A1-0 952 152 beschrieben. Die Passivierung mit der siliciumhaltigen Verbindung ist unabhängig von dem Verformsschritt mit einem Binder, welches bevorzugt Silica ist. Als siliciumhaltigen Verbindungen für die Passivierung der Zeolithoberfläche werden speziell Tetraalkylorthosilikate, besonders Tetraethylorthosilikat, Kieselgele und Polysiloxane beansprucht.

DE-A1-102 19 879 betrifft ein Verfahren zur Herstellung eines Katalysatorträgers bei dem man Zirkoniumdioxidpulver mit einem Bindemittel zu Formkörper formt, trocknet und calciniert, wobei das Bindemittel eine monomere, oligomere oder polymere Organosiliciumverbindung ist. Gegenstand der Anmeldung ist auch der so hergestellte Katalysatorträger selbst, ein Katalysator, der diesen Träger enthält sowie dessen Verwendung als Dehydrierkatalysator.
Gerade im Bereich der Katalysatoren ist es oft erwünscht, nicht das kristalline, katalytisch aktive Material an sich für Umsetzungen einzusetzen, sondern das Material, das in Formkörpern vorliegt. Diese Formkörper sind gerade in vielen großindustriellen Verfahren nötig, um beispielsweise chemische Umsetzungen in beispielsweise Rohrreaktoren oder Rohrbündelreaktoren in z.B. Festbettfahrweise sinnvoll betrieben zu können.

Bekannt ist, dass Katalysatoren und insbesondere Zeolithkatalysatoren mit einem SiO₂-Binder durch Verformung mit kolloidalem Silica erhalten werden können. Die Verwendung von kolloidalem Silica für die Verformung von Zeolithkatalysatoren für die Herstellung von TEDA ist deswegen bevorzugt, weil es eine geringe Azidität aufweist und demzufolge die Bildung von Nebenreaktionen unterdrückt wird (EP-A1-0 831 096; vgl. oben).

US 5614079 betrifft Katalysatoren zum Entwachsen eines Kohlenwasserstoffstroms, wobei die Katalysatoren einen Zeolithen und eine organosiliciumhaltige Verbindung als inertes Bindemittel umfassen.

Erfindungsgemäß wurde erkannt, dass die mechanische Stabilität von Katalysatorformkörpern enthaltend ein zeolithisches Material und mindestens ein siliciumhaltiges Bindemittel für die Verwendung im großtechnischen Maßstab verbesserungswürdig ist, insbesondere dann, wenn die Primärpartikel des zeolithischen Materials klein und kugelförmig sind (z.B. kleiner 1 µm Durchmesser) und/oder wenn die Verformung zu Katalysatorformkörpern mit kommerziell verfügbarem colloidalem Silica (z.B. Ludox®) durchgeführt wird.

Der vorliegenden Erfindung lag die Aufgabe zugrunde, verbesserte Formkörper enthaltend ein zeolithisches Material und mindestens ein siliciumhaltiges Bindemittel, verwendbar als Katalysatoren, bereitzustellen, die eine verbesserte mechanische Stabilität, z.B. gemessen als Schneidhärte (in Newton (N)), z.B. eine Schneidhärte von größer oder gleich 10 N, aufweisen.

Auch sollten die als Katalysatoren in chemischen Umsetzungen, insbesondere bei der Synthese von TEDA, eingesetzten Formkörper bessere Umsätze und Raum-Zeit-Ausbeuten, höhere Selektivitäten und längere Standzeiten ermöglichen.

Bei ihrem Einsatz in der TEDA-Synthese sollte die TEDA-Selektivität größer 90 %, insbesondere bis zu 95 %, betragen und auch bei einem geringen Anteil an Wasser im Feed (z.B. kleiner 90 Gew.-% bezogen auf das Edukt) sollte eine hohe Standzeit (z.B. > 2500 h) erreicht werden.

('Edukt' umfasst ein oder mehrere Amine mit einer Struktureinheit gemäß Formel I, vergl. die Verfahrensansprüche und die Beschreibung unten zur TEDA-Herstellung).

Ganz besonders sollten diese vorteilhaften Eigenschaften eintreten bei Förmkörpern enthaltend ein zeolithisches Material und mindestens ein siliciumhaltiges Bindemittel, in denen mindestens 90 % der Primärpartikel des zeolithischen Materials kugelförmig sind und mindestens 95 Gew.-% der kugelförmigen Primärpartikel einen Durchmesser kleiner oder gleich 1 µm aufweisen.

Überraschenderweise wurde gefunden, dass die Verformung eines zeolithischen Materials (z.B. eines Zeolithpulvers) mit einer siliciumorganischen Verbindung (z.B. einem Silikon) als Bindemittel gelingt und zu Formkörpern führt, deren mechanische Eigenschaften (insbesondere Schneidhärte) den mit kolloidalem Silica verformten Formkörpern deutlich überlegen ist, ohne, dass diese verbesserte mechanische Stabilität sich negativ auf die Selektivität und/oder Aktivität des Formkörpers als Katalysator, z.B. bei der Herstellung von Triethylendiamin (TEDA), auswirkt.

Demgemäss wurde ein Verfahren zur Herstellung eines Formkörpers enthaltend ein zeolithisches Material, wobei das zeolithische Material ein Zeolith vom Pentasil Typ ist, und mindestens ein siliciumhaltiges Bindemittel, umfassend die Schritte
(I) Herstellen eines Gemischs, enthaltend das zeolithische Material, das Bindemittel, ein Anteigmittel und ein Lösungsmittel,
(II) Vermischen und Verdichten des Gemischs,
(III) Verformen des verdichteten Gemischs unter Erhalt eines Formkörpers,
(IV) Trocknen des Formkörpers und
(V) Calcinieren des getrockneten Formkörpers,
gefunden, welches dadurch gekennzeichnet ist, dass als siliciumhaltiges Bindemittel ein cyclisches Silikon der Formel [-SiO(OR)(R')-]ₓ oder ein lineares Silikon der Formel RO-[SiO(OR)(R')-]ₓ-R oder ein Gemisch dieser Silikone eingesetzt wird, wobei R und R'C₁₋₆-Alkylgruppen und x eine Zahl im Bereich von 2 bis 50 bedeuten.

Weiterhin Verfahren zur Herstellung von Triethylendiamin (TEDA) mit den so hergestellten Formkörpern gefunden..

### Die siliciumorganische Verbindung als Bindemittel (Schritt I)

Als siliciumorganisches Bindemittel geeignet sind monomere, oligomere oder polymere Silane, Alkoxysilane, Acyloxysilane, Oximinosilane, Halogensilane, Aminoxysilane, Aminosilane, Amidosilane, Silazane oder Silikone, wie sie beispielsweise in Ullmann's Encyclopedia of Industrial Chemistry, Vol. A24, auf den Seiten 21 bis 56, und in Lehrbuch der Anorganischen Chemie (A.F. Holleman, E. Wiberg), 100. Auflage, Kapitel 2.6.5, auf den Seiten 786 bis 788 beschreiben sind. Insbesondere gehören dazu die monomeren Verbindungen der nachstehenden Formeln (A) bis (F):

(Hal)ₓSiR₄₋ₓ (A)

(Hal)ₓSi(OR)₄₋ₓ (B)

(Hal)ₓSi(NR¹R²)₄₋ₓ (C)

RₓSi(OR¹)₄₋ₓ (D)

RₓSi(NR¹R²)₄₋ₓ (E)

(RO)ₓSi(NR¹R²)₄₋ₓ (F)

worin
- Hal: unabhängig voneinander Halogen (F, Cl, Br oder I, insbesondere Cl),
- R, R¹, R²: unabhängig voneinander H oder einen gegebenenfalls substituierten Alkyl-, Alkenyl-, Alkinyl-, Cycloalkyl-, Cycloalkenyl-, Arylalkyl- oder Arylrest, und
- x: eine ganze Zahl im Bereich von 0 bis 4
bedeuten.

Bei den Alkylresten sind C₁₋₆-Alkylreste bevorzugt. Sie können linear oder verzweigt sein. Bevorzugte Beispiele sind Methyl, Ethyl, n-Propyl, n-Butyl, sec.-Butyl oder tert.-Butyl, speziell Methyl oder Ethyl.

Als Arylreste sind C₆₋₁₀-Arylreste bevorzugt, beispielsweise Phenyl. Bevorzugte Arylalkylreste sind C₇₋₂₀-Arylalkylreste, insbesondere Benzyl. Bevorzugte Alkenylreste sind C₂₋₆-Alkenylreste, insbesondere Vinyl oder Allyl.

Als Alkinylreste sind C₂₋₆-Alkinylreste bevorzugt, beispielsweise Ethinyl oder Propargyl. Bei den Acylresten sind C₂₋₆-Acylreste bevorzugt, insbesondere ein Acetyl. Bevorzugte Cycloalkylreste sind C₅₋₈-Cycloalkylreste, insbesondere Cyclopentyl oder Cyclohexyl.
Bevorzugte Cycloalkenylreste sind C₅₋₈-Cycloalkenylreste, beispielsweise 1-Cyclopentenyl oder 1-Cyclohexenyl.

Beispiele für geeignete Organosiliciumverbindungen der Formel (A) sind SiCl₄, Me-SiCl₃, Me₂SiCl₂ und Me₃SiCl.

Beispiele für geeignete Organosiliciumverbindungen der Formel (B) sind Si(OMe)₄, CiSi(OMe)₃, Cl₂Si(OMe)₂, Cl₃SiOMe. (Me = Methyl)
Beispiele für geeignete Organosiliciumverbindungen der Formel (C) sind Si(NMe₂)₄, ClSi(NMe₂)₃, Cl₂Si(NMe₂)₂, Cl₃SiNMe₂.

Geeignete Organosiliciumverbindungen der Formel (D) sind beispielsweise Si(OEt)₄, MeSi(OEt)₃, Me₂Si(OEt)₂ und Me₃Si(OEt).

Geeignete Verbindungen der Formel (E) sind beispielsweise Me₃Si(N(Me)COMe) und Me₃Si(N(Me)COCH₂C₆H₅).

Eine geeignete Verbindung der Formel (F) ist beispielsweise (MeO)₃Si(NMe₂). Erfindungsgemäß wird als Bindemittel ein cyclisches Silikon der Formel [-SiO(OR)(R')-]ₓ oder ein lineares Silikon der Formel RO-[SiO(OR)(R')-]ₓ-R oder ein Gemisch dieser Silikone eingesetzt, wobei R und R' (unabhängig voneinander) C₁₋₆-Alkylgruppen (wie oben definiert), insbesondere Methyl, Ethyl, und x eine Zahl im Bereich von 2 bis 50, insbesondere eine Zahl im Bereich von 3 bis 20, bedeuten.

Ganz besonders bevorzugte siliciumorganisches Bindemittel sind Methylsilikone, beispielsweise die Silres®-Marken der Fa. Wacker, z.B. Silres® MSE100.

Für die Verformung werden halogenfreie siliciumorganische Bindemittel bevorzugt, um Korrosion während der Präparation der Formkörper bzw. beim Einsatz der Formkörper in der katalytischen Umsetzung zu vermeiden.

Die als Bindemittel eingesetzten Organosiliciumverbindungen sind bevorzugt unter Normalbedingungen flüssig oder werden als Lösung in einem bevorzugt nicht-polaren organischen Lösungsmittel wie Hexan, Toluol und/oder Cyclohexan eingesetzt. Dadurch wird die hochoberflächige mikroporöse Aktivkomponente beim Vermischen gleichmäßig mit der Organosiliciumverbindung benetzt. Beim Calcinieren der Katalysatorformkörper verbrennen die organische Reste des siliciumorganischen Bindemittels.

Dabei wird SiO₂ gebildet, welches im Formkörper sehr fein verteilt vorliegt. Daraus resultiert eine hohe Bindungsverstärkung zwischen den Primärpartikeln der mikroporösen Aktivkomponente und eine sehr gute mechanische Stabilität der erhaltenen Katalysatorformkörper. Durch die Verbrennung der organischen Reste des siliciumorganischen Bindemittels entstehen zusätzliche Poren. Diese Poren sind aufgrund der gleichmäßigen Verteilung des siliciumorganischen Bindemittels im Formkörper ebenfalls sehr gleichmäßig verteilt. Dadurch wird die Gesamtporosität des Katalysatorsträgers erhöht.

Bevorzugt wird durch die Calcinierung der Formkörper gemäß Schritt V mindestens 80 Gew.-%, insbesondere mindestens 95 Gew.-%, der siliciumorganischen Verbindung in hochdisperses SiO₂ umgewandelt. Der Gewichtsanteil des so gebildeten hochdispersen SiO₂ im fertigen Katalysatorformkörper liegt vorzugsweise im Bereich von 5 und 70 Gew.-%, insbesondere im Bereich von 10 und 50 Gew.-%, ganz besonders im Bereich von 10 und 30 Gew.-%.

### Das zeolithische Material (Schritt I)

Das zeolithische Material ist ein Zeolith vom Pentasil Typ und weist bevorzugt die Strukturtypen MFI, MEL oder deren Mischstrukturen auf.

Ganz besonders bevorzugt sind Zeolithe vom Pentasil-Strukturtyp, z.B. ZSM-5, mit einem Alkali- und Erdalkaligehalt von zusammen höchstens 150 Gew.-ppm und insbesondere einem molaren Verhältnis von Si zu Al größer 10, besonders im Bereich von 100 bis 5000, bevorzugt im Bereich von 250 bis 1500, besonders bevorzugt im Bereich von 250 bis 750.

Das zeolithische Material gemäß (I) liegt bevorzugt zumindest teilweise in der H⁺- und/oder NH₄⁺-Form vor. Besonders bevorzugt wird das mikroporöse Material gemäß (I) mindestens teilweise in der H⁺-Form und insbesondere bevorzugt für mehr als 95% in der H⁺-Form eingesetzt.

In einer bevorzugten Ausführungsform sind mindestens 90 Gew.-% der Primärpartikel des zeolithischen Materials kugelförmig und insbesondere mindestens 95 Gew.-% der kugelförmigen Primärpartikel weisen einen Durchmesser im Bereich von kleiner oder gleich 1 µm auf.

Der Begriff "kugelförmig", wie er im Rahmen der vorliegenden Erfindung verwendet wird, bezeichnet Primärpartikel, die bei Untersuchung über Scanning Electron Microscopy (SEM) bei einer Vergrößerung im Bereich von 0,5 • 10⁴ bis 2,0 • 10⁴ im Wesentlichen frei von scharfen Kanten sind. Demgemäss bezeichnet der Begriff "kugelförmig" beispielsweise rein kugelförmige oder deformiert kugelförmig wie beispielsweise elliptische oder quaderförmige Primärteilchen, wobei im Falle der quaderförmigen Primärteilen bei oben erwähnter Untersuchungsmethode im genannten Auflösungsbereich die Kanten abgerundet und nicht scharf sind.

Der Begriff "Strukturtyp ZSM-5", wie er im Rahmen der vorliegenden Erfindung verwendet wird, bezeichnet ein zeolithisches Material, wie es in W.M. Meier, D.H. Olson und Ch. Baerlocher, "Atlas of Zeolithe Structure Types", Elsevier, 5. Auflage, Amsterdam 2001, S. 184-185, als Zeolith vom Strukturtyp ZSM-5 beschrieben ist.

Was die Primärpartikel des zeolithischen Materials anbelangt, so sind Durchmesser von weniger als 1 µm bevorzugt. Weiter bevorzugt sind Durchmesser von höchstens 900 nm, weiter bevorzugt von höchstens 800 nm, weiter bevorzugt von höchstens 700 nm, weiter bevorzugt von höchstens 600 nm und besonders bevorzugt von höchstens 500 nm. Weiter bevorzugt weisen die Primärpartikel des zeolithischen Materials einen Durchmesser im Bereich von mindestens 10 nm, weiter bevorzugt von mindestens 20 nm, weiter bevorzugt von mindestens 30 nm, weiter bevorzugt von mindestens 40 nm und besonders bevorzugt von mindestens 50 nm auf. Die Durchmesser liegen besonders bevorzugt in einem Bereich von 50 bis 500 nm, weiter besonders bevorzugt von 50 bis 400 nm, weiter besonders bevorzugt von 50 bis 300 nm, weiter besonders bevorzugt von 50 bis 250 nm und ganz besonders bevorzugt von 50 bis 200 nm.

Gemäß einer weiteren Ausführungsform der vorliegenden Erfindung kann der Durchmesser auch im Bereich von 50 bis 100 nm oder im Bereich von 100 bis 150 nm oder im Bereich von 150 bis 200 nm oder im Bereich von 200 bis 250 nm liegen.

Die Durchmesser der Primärpartikel, wie sie im Rahmen der vorliegenden Erfindung beschrieben werden, können beispielsweise über die elektronenmikroskopischen Verfahren SEM (Scanning Electron Microscopy) und TEM (Transmission Electron Microscopy) bestimmt werden. Die im Rahmen der vorliegenden Erfindung beschriebenen Durchmesser wurden über SEM bestimmt.

Was das molare Verhältnis von Si zu Al im zeolithischen Material anbelangt, so liegt dieses bevorzugt im Bereich von 100 zu 5000, weiter bevorzugt im Bereich von 250 bis 1500, weiter bevorzugt im Bereich von 250 bis 750 und insbesondere bevorzugt im Bereich von 350 bis 550.

Das bevorzugte kristalline zeolithische Material, insbesondere vom Strukturtyp ZSM-5, weist eine monodisperse Partikelgrößenverteilung auf, wobei der Variationskoeffizient weniger als 50 %, bevorzugt weniger als 25 %, insbesondere weniger als 10 % beträgt. Die Partikelgrößenverteilung wird bestimmt mit Hilfe von Laserbeugungsspektrometrie gemäß DIN 13320.

Die spezifische Oberfläche des bevorzugten kristallinen zeolithischen Materials, bestimmt gemäß DIN 66131 (BET), liegt bevorzugt bei mindestens 350 m²/g und insbesondere bevorzugt bei mindestens 400 m²/g. Beispielsweise liegt die spezifische Oberfläche im Bereich von 350 bis 500 m²/g und insbesondere im Bereich von 400 bis 500 m²/g.

Das Porenvolumen des bevorzugten kristallinen zeolithischen Materials, bestimmt gemäß DIN 66134 (Langmuir; p/pₒ = 0,9995), liegt bevorzugt bei mindestens 0,6 ml/g, besonders bevorzugt bei mindestens 0,7 ml/g und insbesondere bevorzugt bei mindestens 0,8 ml/g. Beispielsweise liegt das Porenvolumen im Bereich von 0,6 bis 1,5 ml/g, weiter bevorzugt im Bereich von 0,7 bis 1,4 ml/g und insbesondere bevorzugt im Bereich von 0,8 bis 1,3 ml/g.

Das bevorzugte zeolithische Material kann generell gemäß sämtlicher geeigneter, dem Fachmann bekannter Verfahren hergestellt werden, die zu dem wie oben spezifizierten zeolithischen Material vom Pentasil-Strukturtyp und insbesondere bevorzugt zum ZSM-5-Strukturtyp führen.

High Silica ZSM-5-Pulver sind beispielsweise kommerziell unter den Namen TZP-9022 (Tricat Zeolites), CBV-28014 (Zeolyst International), T-4573 (Fa. Südchemie), SN-300 (Fa. AlsiPenta), PZ-2/900 (Fa. Uetikon) und P-400 (Fa. UOP) verfügbar.

### Das Lösungsmittel (Schritt I)

Als Lösungsmittel eignen sich z.B. acyclische oder cyclische Ether mit 2 bis 12 Kohlenstoffatomen, wie Diethylether, Di-n-propylether oder dessen Isomere, MTBE, THF, Pyran, oder Lactone, wie gamma-Butyrolacton, Polyether, wie Monoglyme, Diglyme etc., aromatische oder aliphatische Kohlenwasserstoffe, wie Benzol, Toluol, Xylol, Pentan, Cyclopentan, Hexan und Petrolether, oder deren Gemische und besonders auch N-Methylpyrrolidon (NMP) oder Wasser oder wässrige organische Lösungs- oder Verdünnungsmittel der oben genannten Art.

Besonders bevorzugt wird als Lösungsmittel, das auch ein Verdünnungsmittel sein kann, Wasser eingesetzt.

Dem Wasser können sowohl Brönsted-Säuren als auch Brönstedt-Basen zugesetzt werden.

Geeignete Brönstedt-Säuren sind beispielsweise Salzsäure, Schwefelsäure, Phosphorsäure, Salpetersäure oder Carbonsäuren, Dicarbonsäuren oder Oligo- oder Polycarbonsäuren wie beispielsweise Nitrilotriessigsäure, Sulfosalicylsäure oder Ethylendiaminotetraessigsäure.

Geeignete Brönstedt-Basen sind primäre, sekundäre und tertiäre Alkylamine, Ammoniak, sowie Seltenerd-, Alkali- und Erdalkalihydroxide.

Der Anteil an Brönstedt-Säuren bzw. Brönstedt-Basen im Lösungsmittel (z.B. Wasser) liegt besonders zwischen 0,5 und 50 Gew.-%, bevorzugt zwischen 1 und 25 Gew.-%, besonderes bevorzugt zwischen 1 und 10 Gew.-%.

Die Zugabe des Lösungsmittels bewirkt, dass das Gemisch die richtige Konsistenz für die Weiterverarbeitung im Formungsschritt hat. Der Anteil an Lösungsmittel liegt bevorzugt im Bereich von 0,5 bis 80 Gew.-%, weiter bevorzugt im Bereich von 1 bis 50 Gew.-%, weiter bevorzugt im Bereich von 1 bis 40 Gew.-%, und besonders bevorzugt im Bereich von 1 bis 30 Gew.-%, jeweils bezogen auf die Gesamtmasse des Gemisches gemäß Schritt I.

### Das Anteigmittel (Schritt I)

Bei der Herstellung des Gemisches gemäß (I) wird mindestens ein Anteigmittel (= organischer Zusatzstoff) zugegeben.

Als Zusatzstoff (= Anteigmittel) können alle dafür geeigneten Verbindungen verwendet werden. Vorzugsweise sind dies organische, insbesondere hydrophile Polymere wie beispielsweise Cellulose, Cellulosederivate, wie beispielsweise Methylcellulose, Stärke, wie beispielsweise Kartoffelstärke, Polyacrylate, Polymethacrylate, Polyvinylalkohol, Polyvinylpyrrolidon (PVP), Polyisobuten (PIB) oder Polytetrahydrofuran (PTHF).

Insbesondere können als Anteigmittel Verbindungen eingesetzt werden, die auch als Porenbildner wirken.

Das Anteigmittel wird bevorzugt als Feststoff eingesetzt.

Das Anteigmittel wird in einer besonders bevorzugten Ausführungsform des erfindungsgemäßen Verfahrens, wie untenstehend beschrieben, im Schritt V durch Calcinieren zu mindestens 90 Gew.-% entfernt.

Die Zugabe des Anteigmittels bewirkt, dass das Gemisch die richtige Konsistenz für die Weiterverarbeitung im Formungsschritt hat. Der Anteil an Anteigmittel liegt bevorzugt im Bereich von 0,5 bis 80 Gew.-%, weiter bevorzugt im Bereich von 1 bis 50 Gew.-%, weiter bevorzugt im Bereich von 1 bis 40 Gew.-%, und besonders bevorzugt im Bereich von 1 bis 30 Gew.-%, jeweils bezogen auf die Gesamtmasse des Gemisches gemäß Schritt I.

### Porenbildner (optional, Schritt I)

Das Gemisch aus Bindemittel, dem mikroporösen, insbesondere zeolithischen Material, Anteigmittel und Lösungsmittel gemäß I kann zur weiteren Verarbeitung und zur Ausbildung einer plastischen Masse mit mindestens einer weiteren Verbindung versetzt werden. Unter anderem bevorzugt sind hier Porenbildner zu nennen.

Als Porenbildner können im erfindungsgemäßen Verfahren sämtliche Verbindungen eingesetzt werden, die bezüglich des fertigen Formkörpers eine bestimmte Porengröße, eine bestimmte Porengrößenverteilung und/oder bestimmte Porenvolumina bereitstellt.

Bevorzugt werden als Porenbildner im erfindungsgemäßen Verfahren Polymere eingesetzt, die in Wasser oder in wässrigen Lösungsmittelgemischen dispergier-, suspendier- und/oder emulgierbar sind. Bevorzugte Polymere sind hierbei polymere Vinylverbindungen wie beispielsweise Polyalkylenoxide, wie Polyethylenoxide, Polystyrol, Polyacrylate, Polymethacrylate, Polyolefine, Polyamide und Polyester, Kohlenhydrate, wie Cellulose oder Cellulosederivate, wie beispielsweise Methylcellulose, oder Zucker oder Naturfasern. Weitere geeignete Porenbildner sind Pulp oder Graphit.

Bevorzugt sind auch organische saure Verbindungen, die sich in dem Schritt V, wie untenstehend beschrieben, durch Calcinieren entfernen lassen. Zu nennen sind hier Carbonsäuren, insbesondere C₁₋₈-Carbonsäuren, wie beispielsweise Ameisensäure, Oxalsäure und/oder Zitronensäure. Ebenso ist es möglich, zwei oder mehr dieser saueren Verbindungen einzusetzen.

Werden bei der Herstellung des Gemischs gemäß Schritt I Porenbildner eingesetzt, so liegt der Gehalt des Gemischs gemäß (I) an Porenbildner bevorzugt im Bereich von 0,5 bis 80 Gew.-%, bevorzugt im Bereich von 1 bis 50 Gew.-% und besonders bevorzugt im Bereich von 1 bis 30 Gew.-%, jeweils bezogen auf die Menge an mikroporösen, insbesondere zeolithischem Material im Gemisch gemäß (I).

Sollte dies für die zu erzielende Porengrößenverteilung erwünscht sein, kann auch ein Gemisch aus zwei oder mehr Porenbildnern eingesetzt werden.

Die Porenbildner werden in einer besonders bevorzugten Ausführungsform des erfindungsgemäßen Verfahrens, wie untenstehend beschrieben, im Schritt V durch Calcinieren unter Erhalt des porösen Formkörpers zu mindestens 90 Gew.-% entfernt. Gemäß einer bevorzugten Ausführungsform des erfindungsgemäßen Verfahrens werden dabei Formkörper erhalten, die Porenvolumina, bestimmt gemäß DIN 66134, im Bereich von mindestens 0,4 ml/g, bevorzugt im Bereich von 0,4 bis 1,0 ml/g und insbesondere bevorzugt im Bereich von mehr als 0,4 ml/g bis 0,8 ml/g aufweisen.

Die spezifische Oberfläche des erfindungsgemäßen Formkörpers, bestimmt gemäß DIN 66131, liegt bevorzugt bei mindestens 300 m²/g und insbesondere bei mindestens 350 m²/g.

Beispielsweise liegt die spezifische Oberfläche im Bereich von 300 bis 500 m²/g und bevorzugt im Bereich von 350 bis 500 m²/g.

### Mischen und Verdichten (Schritt II)

Die Zugabereihenfolge der Bestandteile zur Herstellung des Gemisches gemäß (I) ist nicht kritisch.

Nach der Herstellung des Gemisches gemäß (I) wird das Gemisch homogenisiert, z.B. für eine Dauer im Bereich von 10 bis 180 Minuten. Besonders bevorzugt werden zur Homogenisierung unter anderem Kneter, Koller oder Extruder eingesetzt. Im kleineren Maßstab wird das Gemisch bevorzugt geknetet. Im industriellen, größeren Maßstab wird zur Homogenisierung bevorzugt gekollert.

Bei der Homogenisierung wird bevorzugt bei Temperaturen im Bereich von ungefähr 10°C bis zum Siedepunkt des Lösungsmittels und Normaldruck oder leichtem überatmosphärischem Druck gearbeitet. Danach kann gegebenenfalls mindestens eine der oben beschriebenen Verbindungen zugegeben werden. Das so erhaltene Gemisch wird solange homogenisiert, vorzugsweise geknetet, bis eine verstrangbare plastische Masse entstanden ist.

Das homogenisierte Gemisch wird in einem folgenden Schritt verformt.

### Verformen des verdichteten Gemischs unter Erhalt eines Formkörpers (Schritt III)

Zur Durchführung dieses Schritts sind solche Verfahren bevorzugt, bei denen die Verformung durch Extrusion in üblichen Extrudern, beispielsweise zu Strängen mit einem Durchmesser von bevorzugt 1 bis 10 mm und besonders bevorzugt 2 bis 5 mm, erfolgt. Derartige Extrusionsvorrichtungen werden beispielsweise in Ullmann's Enzyklopädie der Technischen Chemie, 4. Auflage, Bd. 2, S. 295 ff., 1972, beschrieben.

Neben der Verwendung eines Extruders wird ebenfalls vorzugsweise eine Strangpresse zur Verformung verwendet.

Prinzipiell können jedoch zur Formgebung alle bekannten und/oder geeigneten Knet- und Verformungsvorrichtungen bzw. Verfahren eingesetzt werden. Unter anderem sind hierbei zu nennen:
(i) Brikettieren, d.h. mechanisches Verpressen mit oder ohne Zusatz von zusätzlichem Bindermaterial;
(ii) Pelletieren, d.h. Kompaktieren durch kreisförmige und/oder rotierende Bewegungen;
(iii) Sintern, d.h. das zu verformende Materials wird einer thermischen Behandlung ausgesetzt.

Beispielsweise kann die Formgebung aus der folgenden Gruppe ausgewählt sein, wobei die Kombination von mindestens zwei dieser Methoden explizit eingeschlossen ist: Brikettieren durch Stempelpressen, Walzenpressen, Ringwalzenpressen, Brikettieren ohne Bindemittel; Pelletieren, Schmelzen, Spinning-Techniken, Abscheidung, Schäumen, Sprühtrocknen; Brennen im Schachtofen, Konvektionsofen, Wanderrost, Drehrohrofen, Kollern.

Das Kompaktieren kann bei Umgebungsdruck oder bei gegenüber dem Umgebungsdruck erhöhtem Druck stattfinden, beispielsweise in einem Druckbereich von 1 bar bis zu mehreren hundert bar. Weiterhin kann das Kompaktieren bei Umgebungstemperatur oder bei gegenüber der Umgebungstemperatur erhöhter Temperatur stattfinden, beispielsweise in einem Temperaturbereich von 20 bis 300 °C. Ist Trocknen und/oder Brennen Bestandteil des Formgebungsschritt, so sind Temperaturen bis zu 1500 °C denkbar. Schließlich kann das Kompaktieren in der Umgebungs-Atmosphäre stattfinden oder in einer kontrollierten Atmosphäre. Kontrollierte Atmosphären sind beispielsweise Schutzgas-Atmosphären, reduzierende und/oder oxidierende Atmosphären.

Die Form der erfindungsgemäß hergestellten Formkörper kann beliebig gewählt werden. Insbesondere sind unter anderem Kugeln, ovale Formen, Zylinder oder Tabletten möglich.

Besonders bevorzugt wird im Rahmen der vorliegenden Erfindung das Verformen durch Extrusion des gemäß Schritt II erhaltenen Gemischs durchgeführt, wobei als Extrudate weiter bevorzugt im wesentlichen zylinderförmige Stränge mit einem Durchmesser im Bereich von 0,5 bis 20 mm, bevorzugt im Bereich von 1 bis 10 mm erhalten werden.

Bei den Extrudaten beträgt das Länge : Durchmesser-Verhältnis insbesondere mindestens 2, bevorzugt im Bereich von größer 2 bis 20, besonders bevorzugt im Bereich von 4 bis 10.

### Trocknen des Formkörpers (Schritt IV)

Dem Schritt (III) schließt sich im Rahmen der vorliegenden Erfindung bevorzugt mindestens ein Trocknungsschritt an. Dieser mindestes eine Trocknungsschritt erfolgt dabei bei Temperaturen im Bereich von bevorzugt 80 bis 160 °C, insbesondere von 90 bis 145 °C und besonders bevorzugt von 100 bis 130 °C, wobei die Trocknungsdauer bevorzugt bei 6 h oder mehr, beispielsweise im Bereich von 6 bis 24 h, liegt. Es sind jedoch in Abhängigkeit vom Feuchtigkeitsgehalt des zu trocknenden Materials auch kürzere Trockenzeiten wie beispielsweise ungefähr 1, 2, 3, 4, oder 5 h möglich.

Vor und/oder nach dem Trocknungsschritt kann das bevorzugt erhaltene Extrudat beispielsweise zerkleinert werden. Dabei wird vorzugsweise ein Granulat oder Splitt mit einer Partikelgröße im Bereich von 0,1 bis 5 mm, insbesondere 0,5 bis 2 mm, erhalten.

### Calcinierung des Formkörpers (Schritt V)

Dem Schritt (IV) folgt mindestens ein Calcinierungsschritt. Die Calcinierung wird bei Temperaturen im Bereich von bevorzugt 350 bis 750 °C und insbesondere von 450 bis 600 °C durchgeführt.

Die Calcinierung kann unter jeder geeigneten Gasatmosphäre erfolgen, wobei Luft und/oder Magerluft bevorzugt sind.

Das Calcinieren gemäß (V) kann auch in Gegenwart von Wasserstoff, Stickstoff, Helium, Argon und/oder Dampf oder Gemischen hiervon erfolgen.

Weiter wird die Calcinierung bevorzugt in einem Muffelofen, einem Drehrohrofen und/oder einem Bandkalzinierofen durchgeführt, wobei die Calcinierungsdauer bevorzugt bei 1 h oder mehr, beispielsweise im Bereich von 1 bis 24 h oder im Bereich von 3 bis 12 h liegt. Demgemäß ist es im Rahmen des erfindungsgemäßen Verfahrens beispielsweise möglich, den Formkörper einmal, zweimal oder öfter für jeweils mindestens 1 h, wie beispielsweise jeweils im Bereich von 3 bis 12 h, zu calcinieren, wobei die Temperaturen während eines Calcinierungsschrittes gleich bleiben oder kontinuierlich oder diskontinuierlich geändert werden können. Wird zweimal oder öfter calciniert, können sich die Calcinierungstemperaturen in den einzelnen Schritten unterscheiden oder gleich sein.

Nach dem Calcinierungsschritt kann das calcinierte Material beispielsweise zerkleinert werden. Dabei wird vorzugsweise ein Granulat oder Splitt mit einer Partikelgröße im Bereich von 0,1 bis 5 mm, insbesondere 0,5 bis 2 mm, erhalten.

Die erfindungsgemäß erhaltenen Formkörper weisen Härten auf, die bevorzugt im Bereich von 2 bis 150 N, besonders bevorzugt im Bereich von 5 bis 100 und ganz besonders bevorzugt mindestens 10 N betragen, z.B. im Bereich von 10 bis 75 N liegen.

Die obenstehend beschriebene Härte wurde im Rahmen der vorliegenden Erfindung an einem Apparat der Firma Zwick, Typ BZ2.5/TS1S mit einer Vorkraft von 0,5 N, einer Vorkraftschubgeschwindigkeit von 10 mm/Min. und einer nachfolgenden Prüfgeschwindigkeit von 1,6 mm/Min. bestimmt. Das Gerät besaß einen festsitzenden Drehteller und einen frei beweglichen Stempel mit eingebauter Schneide von 0,3 mm Stärke. Der bewegliche Stempel mit der Schneide war mit einer Kraftmessdose zur Kraftaufnahme verbunden und bewegte sich während der Messung gegen den festsitzenden Drehteller hin, auf der der zu untersuchende Katalysatorformkörper lag. Das Prüfgerät wurde über einen Computer gesteuert, der die Messergebnisse registrierte und auswertete. Die erzielten Werte stellen den Mittelwert aus den Messungen zu jeweils 10 Katalysatorformkörpern dar. Die Katalysatorformkörper wiesen eine zylindrische Geometrie auf, wobei ihre mittlere Länge in etwa dem Zwei- bis Dreifachen des Durchmessers entsprach, und wurden dabei mit der Schneide von 0,3 mm Stärke mit zunehmender Kraft so lange belastet, bis der Formkörper durchtrennt war. Die Schneide wurde dabei senkrecht zur Längsachse des Formkörpers auf den Formkörper aufgebracht. Die dazu benötigte Kraft ist die Schneidhärte (Einheit N).

Nach dem Calcinieren (Schritt V) kann der Formkörper optional mit einer konzentrierten oder verdünnten Broenstedt-Säure oder einem Gemisch aus zwei oder mehr Broenstedt-Säuren behandelt werden. Geeignete Säuren sind beispielsweise Salzsäure, Schwefelsäure, Phosphorsäure, Salpetersäure oder Carbonsäuren, Dicarbonsäuren oder Oligo- oder Polycarbonsäuren, wie beispielsweise Nitrilotriessigsäure, Sulfosalicylsäure oder Ethylendiaminotetraessigsäure.

Diese Behandlung erfolgt in der, optional wässrigen, Flüssigphase bei einer bevorzugten Temperatur im Bereich 10 bis 120 °C und einer bevorzugten Dauer im Bereich von 0,5 bis 12 h.

Bevorzugt schließt sich an diese mindestens eine Behandlung mit mindestens einer Broenstedtsäure mindestens ein Trockenschritt und/oder mindestens ein Calcinierungsschritt an, der jeweils unter den oben beschriebenen Bedingungen durchgeführt wird.

Gemäß einer weiteren bevorzugten Ausführungsform des erfindungsgemäßen Verfahrens können die Katalysatorstränge zur besseren Härtung einer Wasserdampfbehandlung unterzogen werden, nach der bevorzugt nochmals mindestens einmal getrocknet und/oder mindestens einmal calciniert wird. Beispielsweise wird nach mindestens einem Trockenschritt und mindestens einem nachfolgenden Calcinierschritt der calcinierte Formkörper der Wasserdampfbehandlung unterzogen und anschließend nochmals mindestens einmal getrocknet und/oder mindestens einmal calciniert.

### Verwendung der Katalysatorformkörper

Der erfindungsgemäß hergestellte Formkörper kann generell in sämtlichen, dem Fachmann bekannten Verfahren oder Arbeitsschritten eingesetzt werden, in denen die Eigenschaften des Formkörpers und insbesondere des im Formkörper enthaltenen mikroporösen, insbesondere zeolithischen Materials erwünscht sind.

Ganz besonders bevorzugt wird der erfindungsgemäß hergestellte Formkörper als Katalysator bei chemischen Umsetzungen eingesetzt.

Der erfindungsgemäß hergestellte Formkörper kann beispielsweise in der Herstellung von epsilon-Caprolactam aus Cyclohexanonoxim sowie der Herstellung von Ethanolaminen aus Ethylenoxid und Ammoniak eingesetzt werden.

Ganz besonders bevorzugt ist die Verwendung des erfindungsgemäß hergestellten Formkörpers als Katalysator bei der selektiven Synthese von Triethylendiamin (TEDA).

Die vorliegende Erfindung betrifft ein Verfahren zur selektiven Herstellung von Triethylendiamin oder eines alkylsubstituierten Derivats davon durch Umsetzung eines Eduktes, das mindestens eine Struktureinheit (I) 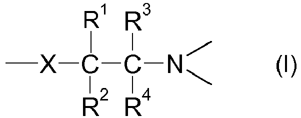 aufweist, wobei R¹, R², R³ und R⁴ unabhängig voneinander ein Wasserstoffatom oder eine Alkylgruppe mit 1 bis 4 Kohlenstoffatomen und X ein Sauerstoff- oder Stickstoffatom darstellen.

Beispiele für solche Verbindungen sind unter anderem Ethylendiamin (EDA), Monoethanolamin, Diethanolamin, Triethanolamin, Piperazin (PIP), Diethylentriamin, Triethylentetramin, Tri(2-aminoethyl)amin, N-(2-aminoethyl)ethanolamin, Morpholin, N-(2-hydroxyethyl)piperazin, N,N'-Bis(2-hydroxyethyl)piperazin, N-(2-aminoethyl)piperazin und N,N'-Bis(2-aminoethyl)piperazin.

Durch die Wahl der Edukte wird die Zusammensetzung des Produktgemischs entscheidend beeinflusst, wobei insbesondere die Vermeidung der Bildung von Nebenprodukten, neben der Verfügbarkeit der Ausgangsprodukte, ein wichtiger Aspekt im Hinblick auf die zu erreichende TEDA-Spezifikation in der Aufarbeitung ist. In den meisten Fällen wird zur Erhöhung der TEDA-Selektivität die Synthese so durchgeführt, dass lediglich ein Teilumsatz des oder der eingesetzten Edukte eintritt. Der Nachteil der geringen Ausbeute wird wegen der erreichbaren niedrigen Mengen an unerwünschten Nebenprodukten in Kauf genommen.

Im Rahmen der vorliegenden Erfindung ist es beispielsweise bevorzugt möglich, TEDA dadurch herzustellen, dass als Edukt Piperazin (PIP) eingesetzt wird. Ebenso ist es möglich, als Edukt Ethylendiamin (EDA) einzusetzen. Möglich ist auch, ein Gemisch aus EDA und PIP als Edukt einzusetzen.

Bevorzugt erfolgt die selektive Herstellung von Triethylendiamin durch Umsetzung eines Eduktes, enthaltend
(A) x Gew.-% Piperazin und
(B) y Gew.-% Ethylendiamin,
wobei x + y = 100 und 0 ≤ x ≤ 100 und 0 ≤ y ≤ 100, am erfindungsgemäßen Formkörper als Katalysator.

Das erfindungsgemäße Verfahren kann diskontinuierlich durchgeführt werden und wird bevorzugt kontinuierlich durchgeführt.

Die erfindungsgemäße Umsetzung kann in der Flüssigphase durchgeführt werden und wird bevorzugt in der Gasphase durchgeführt.

Bevorzugt wird die Umsetzung in Gegenwart mindestens eines Lösungs- oder Verdünnungsmittels durchgeführt.

Als Lösungs- oder Verdünnungsmittel eignen sich z.B. acyclische oder cyclische Ether mit 2 bis 12 Kohlenstoffatomen, wie Dimethylether, Diethylether, Di-n-Propylether oder dessen Isomere, MTBE, THF, Pyran, oder Lactone, wie gamma-Butyrolacton, Polyether, wie Monoglyme, Diglyme etc., aromatische oder aliphatische Kohlenwasserstoffe, wie Benzol, Toluol, Xylol, Pentan, Cyclopentan, Hexan und Petrolether, oder deren Gemische und besonders auch N-Methylpyrrolidon (NMP) oder Wasser oder wässrige organische Lösungs- oder Verdünnungsmittel der oben genannten Art. Weiterhin ist Ammoniak als Lösungs- oder Verdünnungsmittel geeignet.

Besonders bevorzugt wird als Lösungs- oder Verdünnungsmittel, insbesondere Lösungsmittel, Wasser eingesetzt.

Als Verdünnungsmittel bei der Durchführung der Umsetzung in der Gasphase sind auch Inertgase wie Stickstoff (z.B. über die Sättigung des Reaktorzulaufs hinaus) oder Argon geeignet. Bevorzugt wird die Umsetzung in der Gasphase in Gegenwart von Ammoniak durchgeführt.

Die Eduktkomponenten oder der Reaktorzulauf werden vorteilhafterweise vortemperiert.

Als Reaktoren, in denen das erfindungsgemäße Verfahren durchgeführt wird, eignen sich Rührbehälter, insbesondere Rohrreaktoren und Rohrbündelreaktoren.

Der erfindungsgemäße Zeolith-Formkörper, ist im Reaktor bevorzugt als Festbett angeordnet.

Die Umsetzung in der Flüssigphase kann z.B. in der Suspensions-, Riesel- oder Sumpffahrweise erfolgen.

Die bevorzugte Umsetzung in der Gasphase kann in einem Katalysator-Wirbelbett oder bevorzugt -Festbett erfolgen.

Im Falle, dass als Edukt Piperazin (PIP) allein eingesetzt wird, sind Verfahrensführungen bevorzugt, bei denen die Reaktionstemperatur im Bereich von 300 bis 450 °C und besonders bevorzugt im Bereich von 315 bis 400°C liegt. Dabei liegt der Druck, unter dem die Umsetzung stattfindet, in einem Bereich von 0,01 bis 50 bar, bevorzugt im Bereich von 0,5 bis 20 bar und insbesondere bevorzugt im Bereich des Atmosphärendruckes, zuzüglich des Druckverlustes, der beim Durchgang über das Katalysatorbett entsteht.

Piperazin wird weiter besonders bevorzugt im Gemisch mit Wasser eingesetzt, wobei weiter bevorzugt ein Eduktstrom verwendet wird, der mindestens 5 Gew.-% Wasser, bevorzugt 10 bis 70 Gew.-% Wasser und insbesondere 20 bis 70 Gew.-% Wasser enthält, jeweils bezogen auf das Gesamtgewicht des Eduktstroms, enthaltend Piperazin und Wasser.

Im Falle, dass Piperazin als einziges Edukt eingesetzt wird, ist eine WHSV (weight hourly space velocity) im Bereich von 0,01 bis 5 g(PIP) / g(Kat.)•h⁻¹, vorzugsweise im Bereich von 0,02 bis 1 g(PIP) / g(Kat.)•h⁻¹ und besonders bevorzugt im Bereich von 0,05 bis 0,8 g(PIP) / g(Kat.)•h⁻¹ bevorzugt.

Im Falle, dass als Edukt EDA allein eingesetzt wird, sind Verfahrensführungen bevorzugt, bei denen die Reaktionstemperatur im Bereich von 300 bis 400 °C und besonders bevorzugt im Bereich von 315 bis 375 °C liegt. Dabei liegt der Absolutdruck, unter dem die Umsetzung stattfindet, in einem Bereich von 0,01 bis 50 bar, bevorzugt im Bereich von 0,5 bis 20 bar und insbesondere bevorzugt im Bereich des Atmosphärendruckes, zuzüglich des Druckverlustes, der beim Durchgang über das Katalysatorbett entsteht. EDA wird weiter besonders bevorzugt im Gemisch mit Wasser eingesetzt, wobei weiter bevorzugt ein Eduktstrom verwendet wird, der mindestens 5 Gew.-% Wasser, bevorzugt 10 bis 70 Gew.-% Wasser und insbesondere 20 bis 70 Gew.-% Wasser enthält, jeweils bezogen auf das Gesamtgewicht des Eduktstroms, enthaltend EDA und Wasser.

Im Falle, dass EDA als einziges Edukt eingesetzt wird, ist eine WHSV (weight hourly space velocity) im Bereich von 0,01 bis 5 g(EDA) / g(Kat.)•h⁻¹, vorzugsweise im Bereich von 0,02 bis 1 g(EDA) / g(Kat.)•h⁻¹ und besonders bevorzugt im Bereich von 0,05 bis 0,8 g(EDA) / g(Kat.)•h⁻¹ bevorzugt.

Im Falle, dass ein Gemisch aus PIP und EDA als Edukt eingesetzt wird, wird die Reaktion bevorzugt so geführt, dass bei kontinuierlichem Betrieb im stationären Zustand 10 bis 50 Gew.-% Wasser und 90 bis 50 Gew.-% Edukt (Summe der Gew.-%-Anteile der beiden Verbindungen PIP und EDA), weiter bevorzugt 30 bis 50 Gew.-% Wasser und 70 bis 50 Gew.-% Edukt und insbesondere bevorzugt 40 bis 50 Gew.-% Wasser und 60 bis 50 Gew.-% Edukt zugeführt werden, wobei der Anteil des PIP oder des EDA gegebenenfalls zu Gunsten oder zu Lasten des EDA oder des PIP erniedrigt oder erhöht werden kann.

Im Rahmen der wie oben beschriebenen Ausführungsform, gemäß der 35 bis 60 Gew.-%, beispielsweise etwa 40 Gew.-%, EDA zugegeben werden, kann die Umsetzung im stationären Zustand so geführt werden, dass sich EDA im wesentlichen vollständig zu TEDA und PIP umsetzt, wobei PIP dem Produktstrom zusammen mit gegebenenfalls zusätzlich vorhandenen Zwischen- und/oder Nebenprodukten bevorzugt destillativ entzogen wird und, gegebenenfalls nach Abtrennung mindestens eines dieser Zwischen- und/oder Nebenprodukte, mit in etwa derselben Menge an EDA versetzt wird und das resultierende Gemisch, enthaltend EDA und PIP, der Umsetzung wieder zugeführt wird.

Diese Verfahrensvariante wird bevorzugt so durchgeführt, dass der Verbrauch an PIP in der Bilanz gegen Null geht, während des kontinuierlichen Betriebs folglich im Wesentlichen kein zusätzliches PIP zugegeben wird.

Bei dieser Verfahrensführung hat sich überraschend gezeigt, dass die Menge an ausgetragenem EDA gegen Null geht. Die Auftrennung des Reaktoraustrags ist daher besonders einfach.

Ein besonderer Vorteil des Verfahrens besteht darin, dass man Zwischenfraktionen, die sowohl TEDA als auch PIP enthalten, erneut der Umsetzung zuführen kann.

Im Falle, dass ein Gemisch aus EDA und PIP als Edukt eingesetzt wird, sind Verfahrensführungen bevorzugt, bei denen die Reaktionstemperatur im Bereich von 300 bis 450°C, bevorzugt im Bereich von 310 bis 370°C und besonders bevorzugt im Bereich von 310 bis 350°C liegt. Dabei liegt der Absolutdruck, unter dem die Umsetzung stattfindet, in einem Bereich von 0,1 bis 10 bar, bevorzugt im Bereich von 0,8 bis 2 bar und insbesondere bevorzugt im Bereich des Atmosphärendruckes, zuzüglich des Druckverlustes, der beim Durchgang über das Katalysatorbett entsteht.

Im Falle, dass ein Gemisch aus EDA und PIP als Edukt eingesetzt wird, ist eine WHSV (weight hourly space velocity) im Bereich von 0,05 bis 6 g(Edukt) / g(Kat.)•h⁻¹, vorzugsweise im Bereich von 0,2 bis 2 g(Edukt) / g(Kat.)•h⁻¹ und besonders bevorzugt im Bereich von 0,3 bis 1 g(Edukt) / g(Kat.)•h⁻¹ bevorzugt.

Die Verwendung des erfindungsgemäßen Katalysator-Formkörpers, zeichnet sich unter anderem dadurch aus, dass eine sehr hohe Standzeit des Katalysators erreicht wird. Diese liegt bevorzugt im Bereich von mehr als 1000 h, besonders bevorzugt bei mindestens 1200 h, weiter bevorzugt bei mindestens 1400 Stunden, weiter bevorzugt bei mindestens 1600 h, weiter bevorzugt bei mindestens 1800 h und insbesondere bevorzugt bei mindestens 2000 h. Bei konstanten Reaktionsparametern wurde während der oben angegebenen Standzeiten keine Verschlechterung des Reaktionsumsatzes beobachtet.

In einer weiteren Ausführungsform des erfindungsgemäßen Verfahrens wird der Katalysator nach dem Einsatz unabhängig von seiner Form, z.B. nach Abnahme der Aktivität und/oder der Selektivität, durch ein Verfahren regeneriert, bei dem die Regenerierung durch gezieltes Abbrennen der für die Deaktivierung verantwortlichen Beläge erfolgt. Dabei wird bevorzugt in einer Inertgasatmosphäre gearbeitet, die genau definierte Mengen an Sauerstoff liefernden Substanzen enthält. Ein solches Regenerierungsverfahren ist unter anderem in der WO-A-98/55228 und der DE-A1-197 23 949 beschrieben, deren diesbezügliche Offenbarung durch Bezugnahme hiermit vollumfänglich in den Gegenstand der vorliegenden Anmeldung einbezogen wird.

Nach der Regeneration sind die Aktivität und/oder die Selektivität des Katalysators, verglichen mit dem Zustand unmittelbar vor der Regeneration, erhöht.

Der zu regenerierende, erfindungsgemäß eingesetzte Zeolith-Katalysator wird entweder in der Umsetzungsvorrichtung (Reaktor) oder in einem externen Ofen in einer Atmosphäre, die 0,1 bis ungefähr 20 Volumen-Anteile von Sauerstoff liefernden Substanzen, besonders bevorzugt 0,1 bis 20 Volumenanteile Sauerstoff, enthält, auf eine Temperatur im Bereich von 250 °C bis 800°C, vorzugsweise von 400°C bis 550°C und insbesondere von 425°C bis 500°C aufgeheizt. Dabei wird das Aufheizen vorzugsweise mit einer Aufheizrate von 0,1°C/Min. bis 20°C/Min., vorzugsweise von 0,3°C/Min. bis 15°C/Min. und insbesondere von 0,5°C/Min. bis 10°C/Min. durchgeführt.

Während dieser Aufheizphase wird der Katalysator bis zu einer Temperatur aufgeheizt, bei der die sich dort befindlichen, meist organischen Beläge zu zersetzen beginnen, während gleichzeitig die Temperatur über den Sauerstoffgehalt geregelt wird und somit nicht derart ansteigt, dass es zu Schädigungen der Katalysatorstruktur kommt. Das langsame Erhöhen der Temperatur bzw. das Verweilen bei niedriger Temperatur durch Einstellen des entsprechenden Sauerstoffgehaltes und der entsprechenden Heizleistung ist bei hohen organischen Beladungen des zu regenerierenden Katalysators ein wesentlicher Schritt zur Verhinderung einer lokalen Überhitzung des Katalysators.

Sinkt die Temperatur des Abgasstroms am Reaktorausgang trotz steigender Mengen an Sauerstoff liefernden Substanzen im Gasstrom und/oder steigt die Konzentration an Sauerstoff im Reaktionsaustrag auf den Eingangswert an, so ist das Abbrennen der organischen Beläge beendet. Die Dauer der Behandlung beträgt bevorzugt jeweils 1 bis 30 h, vorzugsweise ungefähr 2 bis ungefähr 20 h und insbesondere ungefähr 3 bis ungefähr 10 Stunden.

Das anschließende Abkühlen des so regenerierten Katalysators wird bevorzugt so durchgeführt, dass das Abkühlen nicht zu schnell erfolgt, da sonst die mechanische Festigkeit des Katalysators negativ beeinflusst werden kann.

Es kann erforderlich sein, den Katalysator nach der durchgeführten Regeneration durch Calcinieren, wie oben beschrieben, einer Spülung mit Wasser und/oder verdünnten Säuren wie beispielsweise Salzsäure zu unterziehen, um die durch Verunreinigung der Edukte gegebenenfalls verbleibende anorganische Beladung des Katalysators (Alkalispuren etc.) zu entfernen. Anschließend kann eine erneute Trocknung und/oder ein erneutes Calcinieren des Katalysators durchgeführt werden.

In einer weiteren Ausführungsform des erfindungsgemäßen Verfahrens wird der zumindest teilweise deaktivierte Katalysator vor dem Aufheizen gemäß der Regenerationsprozedur mit einem Lösungsmittel im Umsetzungsreaktor oder in einem externen Reaktor gewaschen, um noch anhaftendes Wertprodukt zu entfernen. Dabei wird das Waschen so durchgeführt, dass zwar die jeweils am Katalysator anhaftenden Wertprodukte von diesem entfernt werden können, aber Temperatur und Druck nicht so hoch gewählt werden, dass die meist organischen Beläge ebenfalls entfernt werden. Vorzugsweise wird der Katalysator dabei mit einem geeigneten Lösungsmittel lediglich gespült. Somit eignen sich für diesen Waschvorgang alle Lösungsmittel, in denen sich das jeweilige Umsetzungsprodukt gut löst. Die benutzte Menge an Lösungsmittel sowie die Dauer des Waschvorgangs sind nicht kritisch. Der Waschvorgang kann mehrmals wiederholt und bei erhöhter Temperatur durchgeführt werden. Bei Verwendung von CO₂ als Lösungsmittel ist überkritischer Druck bevorzugt, ansonsten kann der Waschvorgang unter Normaldruck bzw. erhöhtem oder überkritischem Druck erfolgen. Nach der Beendigung des Waschvorgangs wird der Katalysator im Allgemeinen getrocknet. Obwohl der Trocknungsvorgang im allgemeinen unkritisch ist, sollte die Trocknungstemperatur die Siedetemperatur des zum Waschen verwendeten Lösungsmittels nicht zu stark übersteigen, um ein schlagartiges Verdampfen des Lösungsmittels in den Poren, insbesondere in den Mikroporen zu vermeiden, da auch dies zu Schädigungen des Katalysators führen kann.

Eine bevorzugte Ausführung des Herstellverfahrens besteht darin, dass das erfindungsgemäße, kontinuierliche Verfahren zur Synthese von TEDA bei der Regeneration des erfindungsgemäßen Katalysators nicht unterbrochen werden muss, um so den Verfahrensdurchsatz zu steigern. Dies kann durch die Verwendung von mindestens zwei parallel verschalteten Reaktoren erreicht werden, die wechselweise betrieben werden können.

Die Katalysatorregeneration kann derart durchgeführt werden, dass mindestens einer der parallel geschalteten Reaktoren aus der jeweiligen Reaktionsstufe abgekoppelt wird und der in diesem Reaktor enthaltene Katalysator regeneriert wird, wobei im Laufe des kontinuierlichen Verfahrens in jeder Stufe immer mindestens ein Reaktor zur Umsetzung des Eduktes oder der Edukte zur Verfügung steht.

Das erfindungsgemäß erhaltene TEDA kann zur Verbesserung seiner Reinheit aus geeigneten Lösungsmitteln wie beispielsweise Pentan oder Hexan umkristallisiert werden. Meist ist dies jedoch nicht erforderlich, da TEDA nach dem erfindungsgemäßen Verfahren mit Reinheiten von mindestens 95 Gew.-%, bevorzugt mindestens 96 Gew.-% und besonders bevorzugt von mindestens 97 Gew.-%, hergestellt werden kann.

In einer besonderen Ausgestaltung wird das anspruchsgemäße TEDA-Herstellverfahren kombiniert mit dem sich anschließenden TEDA-Verfahren gemäß EP-A-1 070 717 (BASF AG).

Gemäß dieser Kombination wird zunächst TEDA anspruchsgemäß hergestellt. Bei der sich anschließenden Aufarbeitung des TEDAs (z.B. destillativ), die mehrstufig sein kann, wird das TEDA, bevorzugt in der letzten Aufarbeitungsstufe (insbesondere Destillations- bzw. Rektifikationsstufe), verdampft und das, z.B. am Kopf oder in einem Seitenabzug der Destillationskolonne erhaltene, dampfförmige TEDA, das bevorzugt eine Reinheit von größer 95 Gew.-%, insbesondere von größer 97 Gew.-% besitzt, in ein flüssiges Lösungsmittel einleitet. Diese Einleitung des dampfförmigen TEDAs direkt in ein flüssiges Lösungsmittel wird im Folgenden auch ,TEDA-Quench' genannt.

Die Einleitung des dampfförmigen TEDAs in das flüssige Lösungsmittel erfolgt in einem Quenchapparat, z.B. bevorzugt in einem Fallfilmkondensator (Dünnschicht-, Rieselfilm- oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDAs von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen um eine vollständige Benetzung der Innenwand des Quenchapparates zu erreichen.

Bevorzugt wird die Temperatur im TEDA-Quench durch Temperierung des eingesetzten Lösungsmittels und/oder des Quenchapparates auf 20 bis 100°C, besonders bevorzugt 30 bis 60°C, eingestellt. Der Absolutdruck im TEDA-Quench beträgt bevorzugt 0,5 bis 1,5 bar.

Bevorzugt wird so verfahren, dass, je nach Art des Lösungsmittels, beim TEDA-Quench zunächst Lösungen mit einem TEDA-Gehalt von ca. 1 bis 50 Gew.-%, bevorzugt 20 bis 40 Gew.-%, erhalten werden.

Durch anschließende Auskristallisation des TEDAs aus der so erhaltenen Lösung wird reines TEDA mit hoher Qualität erhalten.

Das flüssige Lösungsmittel wird bevorzugt aus der Gruppe cyclische oder acyclische Kohlenwasserstoffe, chlorierte aliphatische Kohlenwasserstoffe, aromatische Kohlenwasserstoffe, Alkohole, Ketone, aliphatische Carbonsäureester, aliphatische Nitrile und Ether ausgewählt.

Zur Herstellung einer Lösung von reinem TEDA gemäß obiger Verfahrenskombination, die z.B. als Katalysatorlösung bei der Polyurethanschaumherstellung verwendet werden kann, wird als Lösungsmittel für den TEDA-Quench bevorzugt ein Alkohol (z.B. Ethylenglykol, 1,4-Butandiol, bevorzugt Dipropylenglykol) eingesetzt. Die Farbzahl einer so erhaltenen 33 Gew.-%igen TEDA-Lösung in Dipropylenglykol beträgt kleiner 150 APHA, insbesondere kleiner 100 APHA, ganz besonders kleiner 50 APHA.

Die so erhaltenen Lösungen sind bezüglich der Farbzahl im Allgemeinen mehr als 6 Monate, bevorzugt mehr als 12 Monate und insbesondere bevorzugt mehr als 24 Monate lagerstabil.

Zur Herstellung von reinem (kristallinem) TEDA gemäß obiger Verfahrenskombination wird als Lösungsmittel für den TEDA-Quench bevorzugt ein aliphatischer Kohlenwasserstoff, insbesondere ein gesättigter aliphatischer Kohlenwasserstoff mit 5 bis 8 C-Atomen (wie z.B. Hexan, Heptan, bevorzugt Pentan) verwendet. Die Kristallisation des reinen TEDAs aus der erfindungsgemäß hergestellten TEDA-Lösung kann nach den dem Fachmann bekannten Verfahren erfolgen. Die durch eine nachfolgende mehrstufige, oder bevorzugt einstufige, Kristallisation erhaltenen TEDA-Kristalle sind hochrein (Reinheit von im Allgemeinen mindestens 99,5 Gew.-%, insbesondere mindestens 99,8 Gew.-%. Gehalt an PIP kleiner 0,1 Gew.-%, insbesondere kleiner 0,05 Gew.-%, Gehalt an N-Ethylpiperazin kleiner 0,02 Gew.-%, insbesondere kleiner 0,01 Gew.-%) und die Farbzahl einer 33 Gew.-%igen Lösung in Dipropylenglykol beträgt kleiner 50 APHA, insbesondere kleiner 30 APHA.

Alle APHA-Zahlen sind nach DIN ISO 6271 bestimmt.

### Beispiele

Die Herstellung von H-ZSM-5 mit kugelförmigen Zeolithprimärpartikeln und einem Durchmesser < 0,2 µm erfolgte wie in der älteren deutschen Patentanmeldung Nr. 10356184.6 vom 02.12.03 (BASF AG) beschrieben.

Die BET-Oberflächen (m²/g) und die Porenvolumina (ml/g) wurden nach DIN 66131 bzw. DIN 66134 Norm bestimmt.

### GC-Analytik:

Säule RX-5, 30 m; Temperaturprogramm: 80°C - 5°C/Min. - 280°C, Detektor: FID, interner Standard: N-Methylpyrrolidon (NMP).

Die Bestimmung/Messung der Schneidhärte erfolgte wie in der älteren deutschen Patentanmeldung Nr. 10326137.0 vom 06.06.03 (BASF AG) beschrieben:
Die Schneidhärten wurden gemessen an einem Apparat der Firma Zwick (Typ:
BZ2.5/TS1S; Vorkraft: 0,5 N, Vorkraft Geschwindigkeit: 10 mm/Min.; Prüfgeschwindigkeit: 1,6 mm/Min.) und sind die Mittelwerte von jeweils 10 gemessenen Katalysatorsträngen.

Die Schneidhärte wurde im Detail wie folgt bestimmt (siehe auch weiter oben in der Beschreibung):
Extrudate wurden durch eine Schneide von 0,3 mm Stärke mit zunehmender Kraft belastet, bis das Extrudat durchtrennt war. Die dazu benötigte Kraft ist die Schneidehärte in N (Newton). Die Bestimmung erfolgte auf einem Prüfgerät der Firma Zwick, Ulm, mit festsitzendem Drehteller und frei beweglichem, vertikalem Stempel mit eingebauter Schneide von 0,3 mm Stärke. Der bewegliche Stempel mit der Schneide war mit einer Kraftmessdose zur Kraftaufnahme verbunden und bewegte sich während der Messung gegen den festsitzenden Drehteller, auf der das zu messende Extrudat lag. Das Prüfgerät wurde durch einen Computer gesteuert, der die Messergebnisse registrierte und auswertete. Aus einer gut durchmischten Katalysatorprobe wurden 10 gerade, möglichst rissefreie Extrudate mit einer mittleren Länge von 2 bis 3 mal dem Durchmesser entnommen, deren Schneidhärten ermittelt und anschließend gemittelt.
U = Umsatz in Gew.-% bezogen auf der eingesetzte Menge an Ethylendiamin (EDA) und Piperazin (PIP); S = Selektivität der Reaktion für das Triethylendiamin (TEDA) bezogen auf umgesetzte -CH₂-CH₂- -Einheiten stammend aus EDA und PIP.

### Beispiel 1: Herstellung von Katalysator A1 (nicht erfindungsgemäß)

120 g H-ZSM-5 (Modul 1000, Partikelgröße 2-3 µm), wurde bei Raumtemperatur zusammen mit 75 g Ludox® AS40 (collodiales Silica, 39,5 Gew.-% Lösung in ammoniakalischem Wasser), 6 g Methylcellulose und 44 ml Wasser in einem mechanischen Kneter verdichtet. Danach wurde die Paste in einer Strangpresse eingebaut und zu 2 mm Strängen gepresst. Die Stränge wurden in einem Trockenschrank 16 h bei 120°C getrocknet und anschließend in einem Muffelofen 5 h bei 500°C unter Zufuhr von Luftsauerstoff kalziniert.

Die Schneidhärte der Katalysatorformkörper betrug 4 N, die BET-Oberfläche betrug 337 m²/g und das Porenvolumen betrug 0,27 ml/g.

### Beispiel 2: Herstellung von Katalysator A2

130 g H-ZSM-5 (Modul 1000, Partikelgröße 2-3 µm) wurde bei Raumtemperatur zusammen mit 46 g Silres® MSE100 (Methylsilikon, 70 Gew.-% Lösung in Toluol), 6 g Methylcellulose und 55 ml Wasser in einen mechanischen Kneter verdichtet. Die Paste wurde in einer Strangpresse eingebaut und zu 2 mm Strängen gepresst. Danach wurden die Stränge in einem Trockenschrank 16 h bei 120°C getrocknet und anschließend in einem Muffelofen 5 h bei 500°C unter Zufuhr von Luftsauerstoff kalziniert.
Die Schneidhärte der Katalysatorformkörper betrug 21 N, die BET-Oberfläche betrug 388 m²/g und das Porenvolumen betrug 0,23 ml/g.

### Beispiel 3: Herstellung von Katalysator B1 (nicht erfindungsgemäß)

133 g H-ZSM-5 (Modul 1000, Partikelgröße 0,1 - 0,2 µm), wurde bei Raumtemperatur zusammen mit 83 g Ludox® AS40 (collodiales Silica, 39,5 Gew.-% Lösung in ammoniakalischem Wasser), 8 g Methylcellulose und 110 ml Wasser in einem mechanischen Kneter verdichtet. Danach wurde die Paste in einer Strangpresse eingebaut und zu 2 mm Strängen gepresst. Die Stränge wurden in einem Trockenschrank 16 h bei 120°C getrocknet und anschließend in einem Muffelofen 5 h bei 500°C unter Zufuhr von Luftsauerstoff kalziniert.

Die Schneidhärte der Katalysatorformkörper betrug 3 N, die BET-Oberfläche betrug 353 m²/g und das Porenvolumen betrug 0,62 ml/g.

### Beispiel 4: Herstellung von Katalysator B2

128 g H-ZSM-5 (Modul 1000, Partikelgröße 0,1 - 0,2 µm) wurde bei Raumtemperatur zusammen mit 46 g Silres® MSE100 (Methylsilikon, 70 Gew.-% Lösung in Toluol), 6 g Methylcellulose und 120 ml Wasser in einen mechanischen Kneter verdichtet. Die Paste wurde in einer Strangpresse eingebaut und zu 2 mm Strängen gepresst. Danach wurden die Stränge in einem Trockenschrank 16 h bei 120°C getrocknet und anschließend in einem Muffelofen 5 h bei 500°C unter Zufuhr von Luftsauerstoff kalziniert.
Die Schneidhärte der Katalysatorformkörper betrug 20 N, die BET-Oberfläche betrug 445 m²/g und das Porenvolumen betrug 0,60 ml/g.

### Versuchsbeispiel 1: Synthese von TEDA mit Katalysator A1

Für die katalytische Herstellung von Triethylendiamin (TEDA) wurde ein ölbeheiztes Doppelmantelrohr (I = 100 cm, d = 6 mm) mit 20 ml Katalysator A1 (2 mm Stränge) gefüllt und anschließend unter Inertatmosphäre auf 350°C erhitzt. Danach wurde der Katalysator unter drucklosen Bedingungen mit ein Gemisch aus Ethylendiamin (EDA), Piperazin (PIP) und Wasser im Gewichtsverhältnis 25 : 25 : 50 und einer WHSV (weight hourly space velocity) von 1,0 g(Feed) / g(Kat.)•h (= 0,5 g(Edukt) / g(Kat.)•h) belastet. Nach einer Laufzeit von 95 h wurde der Reaktionsaustrag in einem gekühlten Auffangbehälter über einen Zeitraum von 15 Min. gesammelt und die qualitative und quantitative Zusammensetzung der Produkte mit Hilfe von Gaschromatographie bestimmt.

Der Umsatz an EDA betrug 98 % bei einer Selektivität an TEDA von 90 % (siehe Tabelle 1).

### Versuchsbeispiel 2: Synthese von TEDA mit Katalysator A2

Die Durchführung erfolgte analog Versuchbeispiel 1 mit dem Katalysator A2.

Der Umsatz an EDA betrug 95 % bei einer Selektivität an TEDA von 88 % (siehe Tabelle 1).

### Versuchsbeispiel 3: Synthese von TEDA mit Katalysator B1

Die Durchführung erfolgte analog Versuchbeispiel 1 mit dem Katalysator B1.
Der Umsatz an EDA betrug 97 % bei einer Selektivität an TEDA von 93 % (siehe Tabelle 1).

### Versuchsbeispiel 4: Synthese von TEDA mit Katalysator B2

Die Durchführung erfolgte analog Versuchbeispiel 1 mit dem Katalysator B2.
Der Umsatz an EDA betrug 97 % bei einer Selektivität an TEDA von 95 % (siehe Tabelle 1).

**Tabelle 1: Herstellung von TEDA**

| Kat. | SiO₂-Binder | Partikelgröße [µm] | Schneidhärte [N] | U(EDA) [%] | S(TEDA) [%] |
|---|---|---|---|---|---|
| A1 | Ludox | 2-3 | 4 | 98 | 90 |
| A2 | Silres | 2-3 | 21 | 95 | 88 |
| B1 | Ludox | 0,2-0,1 | 3 | 97 | 93 |
| B2 | Silres | 0,2-0,1 | 20 | 97 | 95 |

| | | | | | |
|---|---|---|---|---|---|
| U = Umsatz, S = Selektivität; Zusammensetzung des Reaktionsgemisches jeweils nach 95 h | | | | | |

Durch die Verwendung von Methylsilikon als SiO₂-Binder wird im Vergleich zu der Verwendung von kolloidales Silica als SiO₂-Binder die mechanische Stabilität des Katalysatorformkörpers deutlich verbessert (Vergleich A1 und A2 bzw. B1 und B2). In Kombination mit einer Partikelgröße des ZSM-5-Pulvers von kleiner oder gleich 0,2 µm führt die Verformung des Zeolithpulvers mit einem Methylsilikon zu einer höheren mechanischen Stabilität und eine bessere TEDA-Selektivität bei gleichbleibender Aktivität (Vergleich B1 und B2).

## Patentansprüche

1. Verfahren zur Herstellung eines Formkörpers enthaltend ein zeolithisches Material, wobei das zeolithische Material ein Zeolith vom Pentasil Typ ist, und mindestens ein siliciumhaltiges Bindemittel, umfassend die Schritte
(I) Herstellen eines Gemischs, enthaltend das zeolithische Material, das Bindemittel, ein Anteigmittel und ein Lösungsmittel,
(II) Vermischen und Verdichten des Gemischs,
(III) Verformen des verdichteten Gemischs unter Erhalt eines Formkörpers,
(IV) Trocknen des Formkörpers und
(V) Calcinieren des getrockneten Formkörpers,
**dadurch gekennzeichnet, dass** als siliciumhaltiges Bindemittel ein cyclisches Silikon der Formel [-SiO(OR)(R')-]ₓ oder ein lineares Silikon der Formel RO-[SiO(OR)(R')-]ₓ-R oder ein Gemisch dieser Silikone eingesetzt wird, wobei R und R' C₁₋₆-Alkylgruppen und x eine Zahl im Bereich von 2 bis 50 bedeuten.

2. Verfahren nach Anspruch 1, **dadurch gekennzeichnet, dass** als Bindemittel ein Methylsilikon eingesetzt wird.

3. Verfahren nach einem der vorhergehenden Ansprüche, **dadurch gekennzeichnet, dass** durch die Calcinierung der Formkörper gemäß (V) mindestens 80 Gew.-% der siliciumorganischen Verbindung in hochdisperses SiO₂ umgewandelt wird und der Gewichtsanteil des so gebildeten hochdispersen SiO₂ im Formkörper im Bereich von 5 bis 50 Gew.-% liegt.

4. Verfahren nach dem vorhergehenden Anspruch, **dadurch gekennzeichnet, dass** der Zeolith die Strukturtypen MFI, MEL oder deren Mischstrukturen aufweist.

5. Verfahren nach einem der Ansprüche 1 bis 4, **dadurch gekennzeichnet, dass** das molare Verhältnis von Si zu Al im zeolithischen Material größer 10 beträgt.

6. Verfahren nach einem der Ansprüche 1 bis 4, **dadurch gekennzeichnet, dass** das molare Verhältnis von Si zu Al im zeolithischen Material im Bereich von 100 bis 5000 liegt.

7. Verfahren nach einem der Ansprüche 1 bis 6, **dadurch gekennzeichnet, dass** das zeolithische Material eine spezifische Oberfläche gemäß DIN 66131, BET, von mindestens 350 m²/g aufweist und Poren mit einem Porenvolumen von mindestens 0,6 ml/g gemäß DIN 66134, Langmuir, enthält.

8. Verfahren zur Herstellung von Triethylendiamin (TEDA) oder eines alkylsubstituierten Derivats davon durch Umsetzung eines Eduktes, das mindestens eine Struktureinheit gemäß Formel (I) 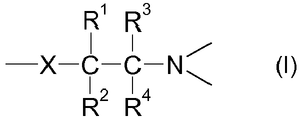 aufweist, wobei R¹, R², R³ und R⁴ unabhängig voneinander ein Wasserstoffatom oder eine Alkylgruppe mit 1 bis 4 Kohlenstoffatomen und X ein Sauerstoff- oder Stickstoffatom darstellen, **dadurch gekennzeichnet, dass** zunächst ein Formkörper nach einem der Ansprüche 1 bis 7 hergestellt wird und anschließend die Umsetzung der Edukte an diesen Formkörper erfolgt.

9. Verfahren nach dem vorhergehenden Anspruch, **dadurch gekennzeichnet, dass** man die Umsetzung kontinuierlich und in der Gasphase durchführt.

10. Verfahren nach einem der beiden vorhergehenden Ansprüche, **dadurch gekennzeichnet, dass** man als Edukt Ethylendiamin und/oder eine oder mehrere Amin-Verbindung/en aus der Gruppe Monoethanolamin, Diethanolamin, Triethanolamin, Piperazin, Diethylentriamin, Triethylentetramin, Tri(2-aminoethyl)amin, Morpholin, N-(2-Aminoethyl)ethanolamin, N-(2-Hydroxyethyl)piperazin, N-(2-Aminoethyl)piperazin, N,N'-Bis(2-Aminoethyl)piperazin, N,N'-Bis(2-Hydroxyethyl)piperazin und N-(2-Aminoethyl)-N'-(2-Hydroxyethyl)piperazin umsetzt.

11. Verfahren nach einem der drei vorhergehenden Ansprüche, **dadurch gekennzeichnet, dass** man als Edukt Ethylendiamin und Piperazinim Gewichtsverhältnis x Piperazin : y Ethylendiamin , wobei x + y = 100 und 0 ≤ x ≤ 100 und 0 ≤ y ≤ 100, umsetzt.

12. Verfahren nach einem der vier vorhergehenden Ansprüche, **dadurch gekennzeichnet, dass** das Edukt in mindestens einem Lösungs- oder Verdünnungsmittel umgesetzt wird.

13. Verfahren nach einem der Ansprüche 8 bis 12 **dadurch gekennzeichnet, dass** die Umsetzung bei einer Temperatur im Bereich von 300 bis 450°C und einem Absolutdruck im Bereich von 0,01 bis 50 bar durchgeführt wird.

## Claims

1. A process for producing a shaped body comprising a zeolitic material, where the zeolitic material is a zeolite of the pentasil type, and at least one silicon-containing binder, which comprises the steps
(I) preparing a mixture comprising the zeolitic material, the binder, a make-up aid and a solvent,
(II) mixing and densifying the mixture,
(III) shaping the densified mixture to give a shaped body,
(IV) drying the shaped body and
(V) calcining the dried shaped body,
wherein the silicon-containing binder used is a cyclic silicone of the formula [-SiO(OR) (R')-]ₓ or a linear silicone of the formula RO-[SiO(OR) (R')-]ₓ-R or a mixture of these silicones, where R and R' are C₁₋₆-alkyl groups and x is in the range from 2 to 50.

2. The process according to claim 1, wherein the binder used is a methylsilicone.

3. The process according to any of the preceding claims, wherein at least 80% by weight of the organosilicon compound is converted into finely divided SiO₂ by the calcination of the shaped bodies in (V) and the proportion by weight of the resulting finely divided SiO₂ in the shaped body is in the range from 5 to 50% by weight.

4. The process according to the preceding claim, wherein the zeolite has the structure types MFI, MEL or a mixed structure derived therefrom.

5. The process according to any of claims 1 to 4, wherein the molar ratio of Si to Al in the zeolitic material is greater than 10.

6. The process according to any of claims 1 to 4, wherein the molar ratio of Si to Al in the zeolitic material is in the range from 100 to 5000.

7. The process according to any of claims 1 to 6, wherein the zeolitic material has a specific surface area in accordance with DIN 66131, BET, of at least 350 m²/g and contains pores having a pore volume of at least 0.6 ml/g in accordance with DIN 66134, Langmuir.

8. A process for preparing triethylenediamine (TEDA) or an alkyl-substituted derivative thereof by reaction of a starting material having at least one structural unit of the formula (I) 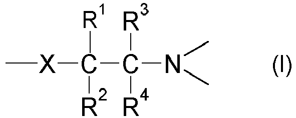 where R¹, R², R³ and R⁴ are each, independently of one another, a hydrogen atom or an alkyl group having from 1 to 4 carbon atoms and X is an oxygen or nitrogen atom, wherein a shaped body according to any of claims 1 to 7 is firstly produced and subsequently the reaction of the starting materials over said shaped body is carried out.

9. The process according to the preceding claim, wherein the reaction is carried out continuously and in the gas phase.

10. The process according to either of the two preceding claims, wherein ethylenediamine and/or one or more amine compound(s) from the group consisting of monoethanolamine, diethanolamine, triethanolamine, piperazine, diethylenetriamine, triethylenetetramine, tri(2-aminoethyl)amine, morpholine, N-(2-aminoethyl)ethanolamine, N-(2-hydroxyethyl)piperazine, N-(2-aminoethyl)piperazine, N,N'-bis(2-aminoethyl)piperazine, N,N'-bis(2-hydroxyethyl)piperazine and N-(2-aminoethyl)-N'-(2-hydroxyethyl)piperazine are reacted as starting material.

11. The process according to any of the three preceding claims, wherein ethylenediamine and piperazine in a weight ratio of x piperazine (PIP): y ethylenediamine (EDA), where x + y = 100 and 0 ≤ x ≤ 100 and 0 ≤ y ≤ 100, are reacted as starting material.

12. The process according to any of the four preceding claims, wherein the starting material is reacted in at least one solvent or diluent.

13. The process according to any of claims 8 to 12, wherein the reaction is carried out at a temperature in the range from 300 to 450°C and an absolute pressure in the range from 0.01 to 50 bar.

## Revendications

1. Procédé de fabrication d'un corps moulé contenant un matériau zéolithique, le matériau zéolithique étant une zéolithe de type pentasile, et au moins un liant contenant du silicium, comprenant les étapes suivantes :
(I) la fabrication d'un mélange, contenant le matériau zéolithique, le liant, un agent d'empâtage et un solvant,
(II) le mélange et le compactage du mélange,
(III) le façonnage du mélange compacté pour obtenir un corps moulé,
(IV) le séchage du corps moulé, et
(V) la calcination du corps moulé séché, **caractérisé en ce qu'**une silicone cyclique de la formule [-SiO(OR)(R')-]ₓ ou une silicone linéaire de la formule RO-[SiO(OR) (R')-]ₓ-R ou un mélange de ces silicones est utilisé en tant que liant contenant du silicium, R et R' signifiant des groupes alkyle en C₁₋₆ et x signifiant un nombre dans la plage allant de 2 à 50.

2. Procédé selon la revendication 1, **caractérisé en ce qu'**une méthylsilicone est utilisée en tant que liant.

3. Procédé selon l'une quelconque des revendications précédentes, **caractérisé en ce qu'**au moins 80 % en poids du composé organique de silicium est transformé en SiO₂ hautement dispersé par la calcination des corps moulés selon (V), et la proportion en poids du SiO₂ hautement dispersé ainsi formé dans le corps moulé se situe dans la plage allant de 5 à 50 % en poids.

4. Procédé selon la revendication précédente, **caractérisé en ce que** la zéolithe présente les types de structure MFI, MEL ou leurs structures mixtes.

5. Procédé selon l'une quelconque des revendications 1 à 4, **caractérisé en ce que** le rapport molaire entre Si et Al dans le matériau zéolithique est supérieur à 10.

6. Procédé selon l'une quelconque des revendications 1 à 4, **caractérisé en ce que** le rapport molaire entre Si et Al dans le matériau zéolithique se situe dans la plage allant de 100 à 5 000.

7. Procédé selon l'une quelconque des revendications 1 à 6, **caractérisé en ce que** le matériau zéolithique présente une surface spécifique selon DIN 66131, BET, d'au moins 350 m²/g, et contient des pores ayant un volume poreux d'au moins 0,6 ml/g selon DIN 66134, Langmuir.

8. Procédé de fabrication de triéthylène-diamine (TEDA) ou d'un dérivé à substitution alkyle de celle-ci par mise en réaction d'un réactif, qui comprend au moins une unité structurale selon la formule (I) : 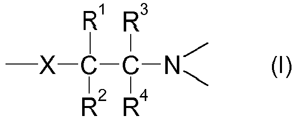 dans laquelle R¹, R², R³ et R⁴ représentent indépendamment les uns des autres un atome d'hydrogène ou un groupe alkyle de 1 à 4 atomes de carbone et X représente un atome d'oxygène ou d'azote, **caractérisé en ce qu'**un corps moulé selon l'une quelconque des revendications 1 à 7 est tout d'abord fabriqué, puis la réaction des réactifs a lieu sur ce corps moulé.

9. Procédé selon la revendication précédente, **caractérisé en ce que** la réaction est réalisée en continu et dans la phase gazeuse.

10. Procédé selon l'une quelconque des deux revendications précédentes, **caractérisé en ce que** de l'éthylène-diamine et/ou un ou plusieurs composés aminés du groupe constitué par la monoéthanolamine, la diéthanolamine, la triéthanolamine, la pipérazine, la diéthylène-triamine, la triéthylène-tétramine, la tri-(2-aminoéthyl)amine, la morpholine, la N-(2-aminoéthyl)éthanolamine, la N-(2-hydroxyéthyl)pipérazine, la N-(2-aminoéthyl)pipérazine, la N,N'-bis(2-aminoéthyl)pipérazine, la N,N'-bis(2-hydroxyéthyl)pipérazine et la N-(2-aminoéthyl)-N'-(2-hydroxyéthyl)pipérazine sont mis en réaction en tant que réactif.

11. Procédé selon l'une quelconque des trois revendications précédentes, **caractérisé en ce que** de l'éthylène-diamine et de la pipérazine sont mis en réaction en tant que réactifs en un rapport en poids de x pipérazine:y éthylène-diamine, avec x+y = 100 et 0 ≤ x ≤ 100 et 0 ≤ y ≤ 100.

12. Procédé selon l'une quelconque des quatre revendications précédentes, **caractérisé en ce que** le réactif est mis en réaction dans au moins un solvant ou diluant.

13. Procédé selon l'une quelconque des revendications 8 à 12, **caractérisé en ce que** la réaction est réalisée à une température dans la plage allant de 300 à 450 °C et à une pression absolue dans la plage allant de 0,01 à 50 bar.
